(19) Europäisches Patentamt
European Patent Office
Office européen des brevets

(11) **EP 3 768 166 B1**

(12) **FASCICULE DE BREVET EUROPEEN**

(45) Date de publication et mention de la délivrance du brevet:
**09.02.2022 Bulletin 2022/06**

(21) Numéro de dépôt: **19715160.8**

(22) Date de dépôt: **06.03.2019**

(51) Classification Internationale des Brevets (IPC):
*A61B 5/024* (2006.01)    *A61B 5/11* (2006.01)
*A61B 5/00* (2006.01)    *G16H 50/30* (2018.01)
*A61B 5/145* (2006.01)

(52) Classification Coopérative des Brevets (CPC):
**A61B 5/14532; A61B 5/024; A61B 5/1118;
A61B 5/4866; A61B 5/7275; A61B 5/746;
G16H 50/30**

(86) Numéro de dépôt international:
**PCT/FR2019/050505**

(87) Numéro de publication internationale:
**WO 2019/180341 (26.09.2019 Gazette 2019/39)**

(54) **SYSTEME DE PREDICTION DE LA GLYCEMIE D'UN PATIENT**

SYSTEM ZUR VORHERSAGE DES BLUTZUCKERSPIEGELS EINES PATIENTEN

SYSTEM FOR PREDICTING A PATIENT'S BLOOD GLUCOSE LEVEL

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **20.03.2018 FR 1852354**

(43) Date de publication de la demande:
**27.01.2021 Bulletin 2021/04**

(73) Titulaire: **Commissariat à l'Energie Atomique et
aux Energies
Alternatives
75015 Paris (FR)**

(72) Inventeurs:
• **ROMERO UGALDE, Hector-Manuel**
**38054 Grenoble Cedex 09 (FR)**
• **BONNET, Stéphane**
**38054 Grenoble Cedex 09 (FR)**
• **DORON, Eléonore Maeva**
**38054 Grenoble Cedex 09 (FR)**

(74) Mandataire: **Cabinet Beaumont
4, Place Robert Schuman
B.P. 1529
38025 Grenoble Cedex 1 (FR)**

(56) Documents cités:
**US-A1- 2007 060 803**    **US-A1- 2014 066 890**
**US-A1- 2014 309 615**    **US-A1- 2016 193 409**

**Description**

La présente demande de brevet revendique la priorité de la demande de brevet français FR18/52354

Domaine

La présente demande concerne le domaine des systèmes de contrôle de glycémie de façon générale, et vise plus particulièrement un système de prédiction de l'évolution future de la glycémie d'un patient.

Exposé de l'art antérieur

On a déjà proposé, par exemple dans la demande de brevet français N°1658881 (B15018/DD16959) déposée le 21 septembre 2016, dans la demande de brevet français N°1658882 (B15267/ DD17175) déposée le 21 septembre 2016, et dans la demande de brevet français N°1756960 (B15860/DD18479) déposée le 21 juillet 2017, des systèmes automatisés de régulation de glycémie, aussi appelés pancréas artificiels, permettant de réguler automatiquement les apports en insuline d'un patient diabétique à partir de son historique de glycémie, de son historique de prise de repas, et de son historique d'injection d'insuline.

Les systèmes de régulation décrits dans les demandes de brevet susmentionnées sont des systèmes de type MPC (de l'anglais "Model-based Prédictive Control"), aussi appelés systèmes à commande prédictive, dans lesquels la régulation de la dose d'insuline administrée tient compte d'une prédiction de l'évolution future de la glycémie du patient, réalisée à partir d'un modèle. Plus particulièrement, dans les systèmes décrits dans les demandes de brevet susmentionnées, le modèle utilisé pour prédire l'évolution future de la glycémie du patient est un modèle physiologique décrivant l'assimilation de l'insuline par le corps du patient et son impact sur la glycémie du patient.

La demande de brevet publiée sous le numéro US 2014/066890 AI divulgue une prédiction du profil de glucose au cours d'un horizon de temps dans le futur proche; le modèle utilisé pour la prédiction est un modèle de type boîte noire tenant compte de la quantité d'insuline embarquée.

De façon plus générale, de nombreuses applications automatisées de contrôle de glycémie utilisent des modèles physiologiques pour prédire l'évolution future de la glycémie d'un patient, et mettre en oeuvre, en tenant compte de cette prédiction, des actions visant à maintenir la glycémie du patient dans une plage souhaitée.

Les modèles physiologiques connus pour la prédiction de l'évolution future de la glycémie d'un patient présentent toutefois certaines limitations. En particulier, dans certaines conditions, il arrive que les prédictions réalisées par les modèles physiologiques connus ne soient pas fiables. Ceci peut conduire à des erreurs lors du contrôle ou lors de régulation de la glycémie du patient, ce qui peut entrainer des risques pour le patient.

Résumé

Ainsi, un mode de réalisation prévoit un système automatisé de contrôle de la glycémie d'un patient, comportant une unité de traitement et de contrôle adaptée à prédire, à partir d'un premier modèle mathématique, l'évolution future de la glycémie $G(t)$ du patient sur une période de prédiction, dans lequel le premier modèle prend pour entrées :

une variable $EE(t)$ représentative de l'évolution, en fonction du temps, de la dépense énergétique du patient ;
une variable $IOB(t)$ représentative de l'évolution, en fonction du temps, de la quantité d'insuline embarquée du patient ; et
une variable $COB(t)$ représentative de l'évolution, en fonction du temps, de la quantité de glucose embarquée du patient,
et dans lequel le premier modèle mathématique est un modèle de type boîte noire, c'est-à-dire un modèle non physiologique défini par la seule observation des effets des variables d'entrée $EE(t)$, $IOB(t)$ et $COB(t)$ sur la variable de sortie $G(t)$, sans tenir compte des différents mécanismes physiologiques connus s'opérant dans le corps du patient.

Selon un mode de réalisation, le premier modèle mathématique est défini comme suit : $G(t) = a.y_G + b.u_{EE} + c.u_{IOB} + d.u_{COB}$, où t est une variable temps discrétisée, $a = [a_1, ..., a_{na}]$ est un vecteur de paramètres de dimension $n_a$, avec $n_a$ entier supérieur ou égal à 1, $b = [b_1, ..., b_{nb}]$ est un vecteur de paramètres de dimension $n_b$, avec $n_b$ entier supérieur ou égal à 1, $c = [c_1, ..., c_{nc}]$ est un vecteur de paramètres de dimension $n_c$, avec $n_c$ entier supérieur ou égal à 1, $d = [d_1, ..., d_{nd}]$ est un vecteur de paramètres de dimension $n_d$, avec $n_d$ entier supérieur ou égal à 1, $y_G = [G(t-1), ..., G(t-n_a)]$ est un vecteur de régression de dimension $n_a$, $u_{EE} = [EE(t-n_{k1}), ..., EE(t-$

$n_{k1}$-$n_b$)] est un vecteur de régression de dimension $n_b$, avec $n_{k1}$ entier supérieur ou égal à 1, $u_{IOB}$ = [IOB(t-$n_{k2}$), ..., IOB(t-$n_{k2}$-$n_c$)] est un vecteur de régression de dimension $n_c$, avec $n_{k2}$ entier supérieur ou égal à 1, et $u_{COB}$ = [COB(t-$n_{k3}$), ..., COB(t-$n_{k3}$-$n_d$)] est un vecteur de régression de dimension $n_d$, avec $n_{k3}$ entier supérieur ou égal à 1.

Selon un mode de réalisation, $n_a$ est compris dans la plage [1,15], $n_b$ est compris dans la plage [1,15], $n_c$ est compris dans la plage [1,15], $n_d$ est compris dans la plage [1,15], $n_{k1}$ est compris dans la plage [1,15], $n_{k2}$ est compris dans la plage [1,15], et $n_{k3}$ est compris dans la plage [1,15].

En fonctionnement, l'unité de traitement et de contrôle reçoit un signal CHO(t) représentatif de l'évolution, en fonction du temps, de la quantité de glucose ingérée par le patient, et un signal I(t) représentatif de l'évolution, en fonction du temps, de la quantité d'insuline injectée au patient. L'unité de traitement et de contrôle est configurée pour calculer les variables d'entrée IOB(t) et COB(t) du premier modèle comme suit :

$$IOB(t) = \sum_{k=0}^{K} I(t-k) * h_{IOB}(k),$$

et

$$COB(t) = \sum_{k=0}^{K} CHO(t-k) * h_{COB}(k),$$

où K est un entier supérieur à 1, k est un entier allant de 0 à K, $h_{IOB}$ est une fonction d'action représentant l'évolution, en fonction du temps, de l'effet de l'insuline injectée sur l'absorption du glucose sanguin, et $h_{COB}$ est une fonction d'action représentant l'évolution, en fonction du temps, de l'effet du glucose ingéré sur la glycémie.

Selon un mode de réalisation, les fonctions d'actions $H_{IOB}$ et $h_{COB}$ sont définies comme suit :

$$h_{IOB}(t) = \left[1 + \frac{t}{\tau_{IOB}}\right] e^{-\frac{t}{\tau_{IOB}}},$$

et

$$h_{COB}(t) = \left[1 + \frac{t}{\tau_{COB}}\right] e^{-\frac{t}{\tau_{COB}}},$$

où $\tau_{IOB}$ et $\tau_{COB}$ sont des constantes de temps.

Selon un mode de réalisation, le système comprend en outre un dispositif de mesure d'une activité physique du patient, la variable d'entrée EE(t) du premier modèle étant calculée par l'unité de traitement et de contrôle à partir de données de sortie PA(t) du dispositif de mesure.

Selon un mode de réalisation, le dispositif de mesure comprend un capteur de mouvements du patient.

Selon un mode de réalisation, le dispositif de mesure comprend en outre un capteur du rythme cardiaque du patient.

Selon un mode de réalisation, le capteur de mouvements fournit un signal $S_{CPM}$ représentatif de mouvements effectués par le patient, et le capteur de rythme cardiaque fournit un signal $S_{HR}$ représentatif du rythme cardiaque du patient, et la variable d'entrée EE(t) du premier modèle est calculée comme suit par l'unité de traitement et de contrôle :

$$EE(t) = \begin{cases} \alpha_1 * S_{HR}(t) + \beta_1, & S_{HR} \geq S1 \\ \alpha_2 * S_{LC}(t) + \beta_2, & S_{HR} < S1 \ et \ S_{LC} < S2, \\ \alpha_3 * S_{LC}(t) + \beta_3, & S_{HR} < S1 \ et \ S_{LC} \geq S2 \end{cases}$$

où le signal $S_{LC}$ est une combinaison linéaire des signaux $S_{HR}$ et $S_{CPM}$, et les grandeurs $\alpha_1$, $\alpha_2$, $\alpha_3$, $\beta_1$, $\beta_2$, $\beta_3$, S1 et S2 sont des paramètres du système.

Selon un mode de réalisation, l'unité de traitement et de contrôle est configurée pour prédire, à partir du premier modèle mathématique, l'évolution future de la glycémie G(t) du patient sur une période de prédiction, déterminer si la glycémie G(t) prédite par le premier modèle reste ou non contenue dans une plage souhaitée, et déclencher une alarme si la glycémie G(t) prédite par le premier modèle sort de la plage souhaitée.

Selon un mode de réalisation, le système comporte en outre un capteur de glycémie, et un dispositif d'injection d'insuline,

l'unité de traitement et de contrôle est adaptée à prédire l'évolution future de la glycémie du patient à partir d'un deuxième modèle mathématique et à commander le dispositif d'injection d'insuline en tenant compte de cette prédiction, et
l'unité de traitement et de contrôle étant configurée pour :

a) mettre en oeuvre une étape de calibration automatique du deuxième modèle en tenant compte d'un historique de glycémie mesurée par le capteur au cours d'une période d'observation passée ;
b) à l'issue de l'étape de calibration, réaliser une première prédiction de l'évolution future de la glycémie du patient à partir du premier modèle et, sur la même période de prédiction, une deuxième prédiction de l'évolution future de la glycémie du patient à partir du deuxième modèle ;
c) calculer au moins un indicateur numérique représentatif de l'écart entre les première et deuxième prédictions ; et
d) en fonction de la valeur de l'indicateur numérique, commander le dispositif d'injection d'insuline en tenant compte de la deuxième prédiction ou commander le dispositif d'injection d'insuline sans tenir compte de la deuxième prédiction.

Selon un mode de réalisation, le deuxième modèle est un modèle physiologique comportant un système d'équations différentielles décrivant l'évolution d'une pluralité de variables d'état en fonction du temps.

Selon un mode de réalisation, l'étape a) de calibration automatique du deuxième modèle comprend une étape d'estimation de paramètres du système d'équations différentielles par minimisation d'une grandeur représentative de l'erreur, pendant une période d'observation passée, entre la glycémie estimée à partir du deuxième modèle physiologique et la glycémie mesurée par le capteur.

Brève description des dessins

Ces caractéristiques et leurs avantages, ainsi que d'autres, seront exposés en détail dans la description suivante de modes de réalisation particuliers faite à titre non limitatif en relation avec les figures jointes parmi lesquelles :

la figure 1 représente de façon schématique, sous forme de blocs, un exemple d'un système automatisé de régulation de la glycémie d'un patient ;
la figure 2 est une représentation simplifiée d'un modèle physiologique utilisé dans le système de la figure 1 pour prédire l'évolution future de la glycémie du patient ;
la figure 3 est un diagramme illustrant un exemple d'un procédé automatisé de régulation de glycémie pouvant être mis en oeuvre par le système de la figure 1 ;
la figure 4 représente de façon schématique, sous forme de blocs, un exemple d'un mode de réalisation d'un système automatisé de prédiction de la glycémie d'un patient ;
la figure 5 est une représentation simplifiée d'un modèle utilisé dans le système de la figure 4 pour prédire l'évolution future de la glycémie du patient ;
la figure 6 est un diagramme illustrant un exemple d'un procédé de construction du modèle de la figure 5 ;
la figure 7 est un diagramme illustrant un exemple d'un procédé automatisé de contrôle de glycémie pouvant être mis en oeuvre par le système de la figure 4 ; et
la figure 8 est un diagramme illustrant un autre exemple d'un procédé automatisé de contrôle de glycémie pouvant être mis en oeuvre par le système de la figure 4.

Description détaillée

De mêmes éléments ont été désignés par de mêmes références dans les différentes figures. Par souci de clarté, seuls les éléments utiles à la compréhension des modes de réalisation décrits ont été représentés et sont détaillés. En particulier, la réalisation matérielle de l'unité de traitement et de contrôle des systèmes décrit n'a pas été détaillée, la réalisation d'une telle unité de traitement et de contrôle étant à la portée de l'homme du métier à partir des indications fonctionnelles de la présente description. Sauf précision contraire, les expressions "approximativement", "sensiblement", et "de l'ordre de" signifient à 10 % près, de préférence à 5 % près.

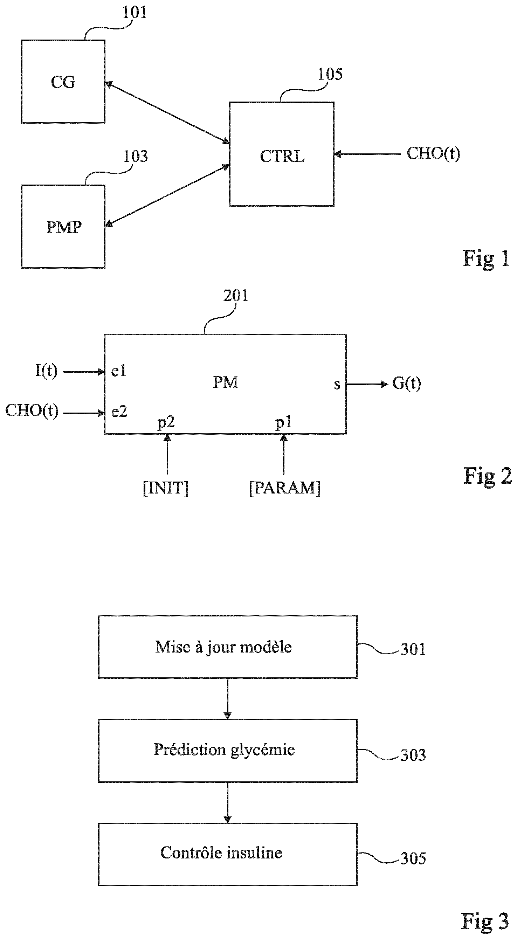

La figure 1 représente de façon schématique, sous forme de blocs, un exemple d'un système automatisé de régulation de la glycémie d'un patient du type décrit dans les demandes de brevet français N°1658881, N°1658882 et N°1756960 susmentionnées.

Le système de la figure 1 comprend un capteur 101 (CG) adapté à mesurer la glycémie du patient. En fonctionnement normal, le capteur 101 peut être positionné à demeure sur ou dans le corps du patient, par exemple à hauteur de son abdomen. Le capteur 101 est par exemple un capteur de type CGM (de l'anglais "Continuous Glucose Monitoring" - surveillance continue de glycémie), c'est-à-dire un capteur adapté à mesurer en continu, par exemple de façon périodique (par exemple au moins une fois toutes les cinq minutes) la glycémie du patient. Le capteur 101 est par exemple un capteur de glycémie en sous-cutané.

Le système de la figure 1 comprend en outre un dispositif d'injection d'insuline 103 (PMP), par exemple un dispositif d'injection en sous-cutané. Le dispositif 103 est par exemple un dispositif d'injection automatique de type pompe à insuline, comportant un réservoir d'insuline relié à une aiguille d'injection implantée sous la peau du patient, la pompe pouvant être commandée électriquement pour injecter automatiquement des doses d'insuline déterminées à des instants déterminés. En fonctionnement normal, le dispositif d'injection 103 peut être positionné à demeure dans ou sur le corps du patient, par exemple au niveau de son abdomen.

Le système de la figure 1 comprend en outre une unité de traitement et de contrôle 105 (CTRL) reliée d'une part au capteur de glycémie 101, par exemple par liaison filaire ou par liaison radio (sans fil), et d'autre part au dispositif d'injection 103, par exemple par liaison filaire ou radio. En fonctionnement, l'unité de traitement et de contrôle 105 est adaptée à recevoir les données de glycémie du patient mesurées par le capteur 101, et à commander électriquement le dispositif 103 pour injecter au patient des doses d'insuline déterminées à des instants déterminés (par exemple bolus et basal). Dans cet exemple, l'unité de traitement et de contrôle 105 est en outre adaptée à recevoir, par l'intermédiaire d'une interface utilisateur non détaillée, des données CHO(t) représentatives de l'évolution, en fonction du temps, de la quantité de glucose ingérée par le patient.

L'unité de traitement et de contrôle 105 est adaptée à déterminer les doses d'insuline à injecter au patient en tenant compte notamment de l'historique de glycémie mesurée par le capteur 101, de l'historique d'insuline injectée par le dispositif 103, et de l'historique d'ingestion de glucose par le patient. Pour cela, l'unité de traitement et de contrôle 105 comprend un circuit de calcul numérique (non détaillé), comprenant par exemple un microprocesseur. L'unité de traitement et de contrôle 105 est par exemple un dispositif mobile porté par le patient tout au long de la journée et/ou de la nuit, par exemple un dispositif de type smartphone configuré pour mettre en oeuvre un procédé de régulation du type décrit ci-après.

Dans l'exemple de la figure 1, l'unité de traitement et de contrôle 105 est adaptée à déterminer la quantité d'insuline à administrer au patient en tenant compte d'une prédiction de l'évolution future de sa glycémie en fonction du temps. Plus particulièrement, l'unité de traitement et de contrôle 105 est adaptée, à partir de l'historique d'insuline injectée et de l'historique de glucose ingéré, et en se basant sur un modèle physiologique décrivant l'assimilation de l'insuline par le corps du patient et son impact sur la glycémie, à déterminer une courbe représentative de l'évolution attendue de la glycémie du patient en fonction du temps, sur une période à venir appelée période de prédiction ou horizon de prédiction, par exemple une période de 1 à 10 heures. En tenant compte de cette courbe, l'unité de traitement et de contrôle 105 détermine les doses d'insuline qu'il conviendrait d'injecter au patient pendant la période de prédiction à venir, pour que la glycémie réelle (par opposition à la glycémie estimée à partir du modèle physiologique) du patient reste dans des limites acceptables, et en particulier pour limiter les risques d'hyperglycémie ou d'hypoglycémie. Dans ce mode de fonctionnement, comme cela sera expliqué plus en détail ci-après, les données de glycémie réelle mesurées par le capteur 101 sont utilisées principalement à des fins de calibration du modèle physiologique.

La figure 2 est une représentation simplifiée d'un modèle physiologique 201 (PM) utilisé dans le système de la figure 1 pour prédire l'évolution future de la glycémie du patient. Sur la figure 2, le modèle est représenté sous la forme d'un bloc de traitement comportant :

une entrée e1 sur laquelle est appliquée un signal I(t) représentatif de l'évolution, en fonction du temps t, de la quantité d'insuline injectée au patient ;
une entrée e2 sur laquelle est appliquée un signal CHO(t) représentatif de l'évolution, en fonction du temps t, de la quantité de glucose ingérée par le patient ; et
une sortie s fournissant un signal G(t) représentatif de l'évolution, en fonction du temps t, de la glycémie du patient.

Le modèle physiologique 201 est par exemple un modèle compartimentai comportant, outre les variables d'entrée I(t) et CHO(t) et la variable de sortie G(t), une pluralité de variables d'état correspondant à des variables physiologiques du patient, évoluant en fonction du temps. L'évolution temporelle des variables d'état et de la variable de sortie G(t) est régie par un système d'équations différentielles comportant une pluralité de paramètres représentés sur la figure 2 par un vecteur [PARAM] appliqué sur une entrée p1 du bloc 201. La réponse du modèle physiologique est en outre conditionnée par les états initiaux ou valeurs initiales affectés aux variables d'état, représentés sur la figure 2 par un vecteur [INIT] appliqué sur une entrée p2 du bloc 201.

A titre d'exemple, le modèle physiologique 201 utilisé dans le système de la figure 1 est le modèle dit de Hovorka, décrit dans l'article intitulé "Nonlinear model prédictive control of glucose concentration in subjects with type 1 diabetes" de Roman Hovorka et al. (Physiol Meas. 2004;25:905-920), et dans l'article intitulé "Partitioning glucose distribution/transport, disposai, and endogenous production during IVGTT", de Roman Hovorka et al. (Am J Physiol Endocrinol Metab 282: E992-E1007, 2002). Plus généralement, tout autre modèle physiologique décrivant l'assimilation de l'insuline par le corps d'un patient et son effet sur la glycémie du patient peut être utilisé.

Parmi les paramètres du vecteur [PARAM], certains peuvent être considérés comme constants pour un patient donné. D'autres paramètres, appelés ci-après paramètres temps-dépendants, sont en revanche susceptibles d'évoluer dans le temps. Du fait de cette variabilité de certains paramètres du système, il est en pratique nécessaire de ré-étalonner ou re-calibrer régulièrement le modèle en cours d'utilisation, par exemple toutes les 1 à 20 minutes, par exemple toutes les 5 minutes, pour s'assurer que les prédictions du modèle restent pertinentes. Cette mise à jour du modèle, aussi appelée personnalisation du modèle, doit pouvoir être réalisée de façon automatique par le système de la figure 1, c'est-à-dire sans qu'il soit nécessaire de mesurer physiquement les paramètres temps-dépendants du système sur le patient puis de les transmettre à l'unité de traitement et de contrôle 105.

La figure 3 est un diagramme illustrant un exemple d'un procédé automatisé de régulation de glycémie pouvant être mis en oeuvre par le système de la figure 1.

Ce procédé comprend une étape 301 de re-calibration ou mise à jour du modèle, pouvant par exemple être répétée à intervalles réguliers, par exemple toutes les 1 à 20 minutes. Lors de cette étape, l'unité de traitement et de contrôle 105 met en oeuvre un procédé de ré-estimation des paramètres temps-dépendants du modèle en tenant compte des données d'insuline effectivement injectée par le dispositif 103 et des données de glycémie réelle mesurée par le capteur 101 pendant une période d'observation passée de durée $\Delta T$, par exemple une période de 1 à 10 heures précédant l'étape de calibration. Plus particulièrement, lors de l'étape de calibration, l'unité de traitement et de contrôle 105 simule le comportement du patient sur la période d'observation passée à partir du modèle physiologique (en tenant compte des éventuelles ingestions de glucose et injections d'insuline pendant cette période), et compare la courbe de glycémie estimée par le modèle à la courbe de glycémie réelle mesurée par le capteur pendant cette même période. L'unité de traitement et de contrôle 105 recherche alors, pour les paramètres temps-dépendants du modèle, un jeu de valeurs conduisant à minimiser une grandeur représentative de l'erreur entre la courbe de glycémie estimée par le modèle et la courbe de glycémie réelle pendant la période d'observation. A titre d'exemple, l'unité de traitement et de contrôle recherche un jeu de paramètres conduisant à minimiser un indicateur m représentatif de l'aire ou écart entre la courbe de glycémie estimée par le modèle et la courbe de glycémie réelle pendant la période d'observation, aussi appelé écart quadratique moyen entre la glycémie estimée et la glycémie réelle, par exemple défini comme suit :

$$m = \frac{1}{\Delta T}\sum_{t=t_0-\Delta T}^{t_0}|G^r(t)-G(t)|^2 \qquad (\text{eq1})$$

où t est une variable temps discrétisée, $t_0$-$\Delta T$ correspond à l'instant de début de la phase d'observation passé, $t_0$ correspond à l'instant de fin de la phase d'observation passée (correspondant par exemple à l'instant de début de l'étape de calibration du modèle), $G^r$ est la courbe d'évolution temporelle de la glycémie réelle mesurée par le capteur 101 pendant la période $[t_0$-$\Delta T, t_0]$, et G est la courbe de glycémie estimée à partir du modèle pendant la période $[t_0$-$\Delta T, t_0]$. A titre de variante, pour le calcul de l'écart quadratique moyen, la variable $\Delta T$ peut être remplacée par le nombre de mesures réalisées pendant la période d'observation passée. L'algorithme de recherche de paramètres optimaux utilisé lors de cette étape n'est pas détaillé dans la présente demande, les modes de réalisation décrits étant compatibles avec les algorithmes usuels utilisés dans des domaines variés pour résoudre des problèmes d'optimisation de paramètres par minimisation d'une fonction de coût (avec ou sans contraintes).

On notera que lors de l'étape 301, outre les paramètres temps-dépendants du modèle, l'unité de traitement et de contrôle 105 définit un vecteur [INIT] d'états initiaux (états à l'instant $t_0$-$\Delta T$) des variables d'état du modèle, pour pouvoir simuler le comportement du patient à partir du modèle. Pour définir les états initiaux des variables d'état du modèle, une première possibilité consiste à faire l'hypothèse que, dans la période précédant la période d'observation $[t_0$-$\Delta T, t_0]$ sur laquelle est basée la calibration du modèle, le patient se trouvait dans un état stationnaire, avec un débit d'insuline injectée constant, et une prise alimentaire de glucose nulle. Sous cette hypothèse, toutes les dérivées du système d'équations différentielles peuvent être considérées comme nulles à l'instant initial $t_0$-$\Delta T$. Les valeurs à l'instant $t_0$-$\Delta T$ des variables d'état du système peuvent alors être calculées analytiquement. Pour améliorer l'initialisation, une autre possibilité consiste à faire les mêmes hypothèses que précédemment, mais en ajoutant la contrainte que la glycémie estimée à l'instant $t_0$-$\Delta T$ soit égale à la glycémie réelle mesurée par le capteur. Pour améliorer encore l'initialisation, une autre possibilité est de considérer les états initiaux des variables d'état du modèle comme des variables aléatoires, au même titre que les paramètres temps-dépendants du modèle. Les états initiaux des variables d'état sont alors déterminés de la même façon que les paramètres temps-dépendants du modèle, c'est-à-dire que l'unité de traitement et de contrôle 105 recherche un jeu de valeurs d'états initiaux [INIT] conduisant à minimiser une grandeur représentative de l'erreur entre la courbe de glycémie estimée par le modèle et la courbe de glycémie réelle pendant la période d'observation passée.

Le procédé de la figure 3 comprend en outre, après l'étape 301, une étape 303 de prédiction, par l'unité de traitement et de contrôle 105, de l'évolution temporelle de la glycémie du patient sur une période de prédiction à venir $[t0, t_0+T_{pred}]$ de durée $T_{pred}$, par exemple comprise entre 1 et 10 heures, à partir du modèle physiologique mis à jour à l'étape 301 et en tenant compte de l'historique d'insuline injectée au patient et de l'historique de glucose ingéré par le patient.

Le procédé de la figure 3 comprend de plus, après l'étape 303, une étape 305 de détermination, par l'unité de traitement et de contrôle 105, en tenant compte de la courbe de glycémie future prédite à l'étape 303, des doses d'insuline à injecter au patient pendant la période de prédiction à venir $[t0,t_0+T_{pred}]$. A l'issue de cette étape, l'unité de traitement et de contrôle 105 peut programmer le dispositif d'injection 103 pour administrer les doses déterminées pendant la période de prédiction $[t0,t_0+T_{pred}]$.

Les étapes 303 de prédiction de la glycémie et 305 et détermination des doses futures d'insuline à administrer peuvent par exemple être réitérées à chaque mise à jour du modèle physiologique (c'est-à-dire après chaque itération de l'étape 301), à chaque nouvelle ingestion de glucose signalée par le patient, et/ou à chaque nouvelle administration d'une dose d'insuline par le dispositif d'injection 103.

Dans un système de régulation du type décrit en relation avec les figures 1 à 3, la fiabilité de la prédiction de l'évolution future de la glycémie est particulièrement importante pour déterminer correctement les doses d'insuline à administrer au patient, et par conséquent pour réguler correctement la glycémie du patient.

Plus généralement, dans de nombreuses applications de contrôle automatisé de glycémie, la prédiction de l'évolution future de la glycémie du patient joue un rôle important pour permettre le maintien de la glycémie du patient dans une plage souhaitée (correspondant par exemple à une plage de normoglycémie) .

Comme indiqué précédemment, les modèles physiologiques connus pour la prédiction de l'évolution future de la glycémie d'un patient présentent toutefois certaines limitations. En particulier, dans certaines conditions, il arrive que les prédictions réalisées par ces modèles ne soient pas fiables. Ceci peut conduire à des erreurs lors du contrôle ou lors de régulation de la glycémie du patient, ce qui peut entrainer des risques pour le patient.

Ainsi, il serait souhaitable de pouvoir disposer d'un système automatisé de prédiction de glycémie palliant tout ou partie des inconvénients des systèmes de prédiction de glycémie connus.

Selon un aspect des modes de réalisation décrits, on prévoit un système dans lequel la prédiction de l'évolution future de la glycémie du patient est réalisée en tenant compte non seulement de l'historique d'insuline injectée au patient et de l'historique de glucose ingéré par le patient, mais aussi d'une donnée représentative de l'activité physique du patient.

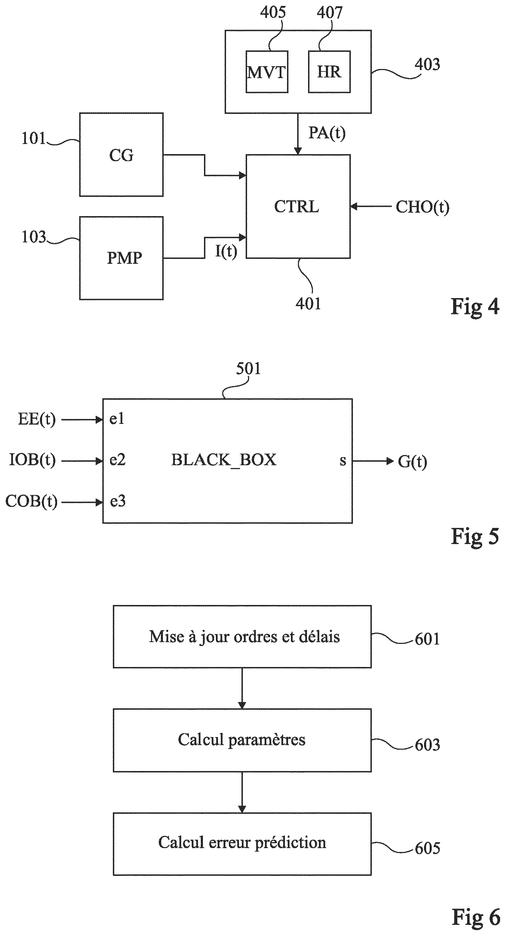

La figure 4 représente de façon schématique, sous forme de blocs, un exemple d'un mode de réalisation d'un système automatisé de prédiction de la glycémie d'un patient.

Le système de la figure 4 comprend une unité de traitement et de contrôle 401 (CTRL). En fonctionnement, l'unité de traitement et de contrôle 401 est adaptée à recevoir des données CHO(t) représentatives de l'évolution, en fonction du temps, de la quantité de glucose ingérée par le patient, des données I(t) représentatives de l'évolution, en fonction du temps, de la quantité d'insuline injectée au patient, et des données PA(t) représentatives de l'évolution, en fonction du temps, de l'activité physique du patient.

Les données de glucose ingéré CHO(t) sont par exemple saisies par le patient par l'intermédiaire d'une interface utilisateur non détaillée.

Les données d'insuline injectée I(t) peuvent également être saisies par le patient par l'intermédiaire d'une interface utilisateur. A titre de variante, les données d'insuline injectées I(t) sont directement communiquées à l'unité de traitement et de contrôle 401 par un dispositif d'injection d'insuline. Dans l'exemple de la figure 4, le système comprend un dispositif d'injection d'insuline 103 (PMP), par exemple identique ou similaire au dispositif 103 de la figure 1. Le dispositif d'injection d'insuline 103 est relié à l'unité de traitement et de contrôle 401, par exemple par liaison filaire ou par liaison radio (sans fil), et communique à l'unité de traitement et de contrôle 401 les données d'insuline injectée I(t). A titre de variante, l'unité de traitement et de contrôle 105 est configurée pour commander électriquement le dispositif 103 pour injecter au patient des doses d'insuline déterminées à des instants déterminés. Dans ce cas, les données d'insuline injectée I(t) sont connues de l'unité de traitement et de contrôle 401 et n'ont donc pas besoin de lui être transmises.

Dans l'exemple de la figure 4, le système comprend en outre un dispositif 403 adapté à mesurer l'activité physique du patient. Le dispositif 403 est reliée à l'unité de traitement et de contrôle 401, par exemple par liaison filaire ou par liaison radio (sans fil), et communique à l'unité de traitement et de contrôle 401 les données d'activité physique PA(t) du patient. Le dispositif 403 est par exemple un dispositif mobile porté par le patient tout au long de la journée et/ou de la nuit. A titre d'exemple, le dispositif 403 comprend au moins un capteur de mouvements 405 (MVT), par exemple un accéléromètre. Le dispositif 403 peut en outre comprendre un capteur 407 (HR) du rythme cardiaque du patient.

Le système de la figure 4 peut de plus comporter un capteur 101 (CG), par exemple identique ou similaire au capteur 101 du système de la figure 1, adapté à mesurer la glycémie du patient. Le capteur 101 peut être relié, par exemple par liaison filaire ou par liaison radio (sans fil), à l'unité de traitement et de contrôle 401. A titre d'exemple, en fonctionnement, l'unité de traitement et de contrôle 401 est adaptée à recevoir les données de glycémie du patient mesurées par le capteur 101.

Dans le mode de réalisation de la figure 4, l'unité de traitement et de contrôle 401 est adaptée à prédire l'évolution future de la glycémie du patient, et, éventuellement, à mettre en oeuvre une action de contrôle ou de régulation de la glycémie du patient, dans le but de maintenir la glycémie du patient dans une plage souhaitée. Plus particulièrement, l'unité de traitement et de contrôle 401 est adaptée, à partir de l'historique d'insuline injectée, de l'historique de glucose ingéré, et de l'historique d'activité physique du patient, et en se basant sur un modèle mathématique, à déterminer une courbe représentative de l'évolution attendue de la glycémie du patient en fonction du temps, sur une période à venir. Pour cela, l'unité de traitement et de contrôle 401 comprend un circuit de calcul numérique (non détaillé), comprenant par exemple un microprocesseur. L'unité de traitement et de contrôle 401 est par exemple un dispositif mobile porté par le patient tout au long de la journée et/ou de la nuit, par exemple un dispositif de type smartphone configuré pour mettre en oeuvre un procédé de prédiction et/ou de contrôle de la glycémie du patient.

La figure 5 est une représentation simplifiée d'un modèle mathématique 501 (BLACK BOX) utilisé dans le système de la figure 4 pour prédire l'évolution future de la glycémie du patient. Sur la figure 5, le modèle est représenté sous la forme d'un bloc de traitement comportant :

une entrée e1 sur laquelle est appliqué un signal EE(t) représentatif de l'évolution, en fonction du temps t, de la dépense énergétique du patient, c'est-à-dire de l'effort physique réalisé par le patient à l'instant t (à titre d'exemple, le signal EE(t) est représentatif de la quantité d'énergie dépensée à l'instant t par le patient pour permettre à son organisme de fonctionner correctement, d'assurer toutes ses fonctions physiques, et, le cas échéant, de réaliser un effort physique) ;
une entrée e2 sur laquelle est appliqué un signal IOB(t) représentatif de l'évolution, en fonction du temps t, de la quantité d'insuline embarquée ("insulin on board" en anglais) du patient, c'est-à-dire de la quantité d'insuline encore active (c'est-à-dire encore susceptible d'avoir un effet sur la glycémie) à l'instant t dans le corps du patient ;
une entrée e3 sur laquelle est appliqué un signal COB(t) représentatif de l'évolution, en fonction du temps t, de la quantité de glucose embarquée ("carbohydrate on board" en anglais) du patient, c'est-à-dire de la portion de la quantité de glucose ingérée par le patient encore susceptible, à l'instant t, d'avoir un effet sur la glycémie du patient ; et
une sortie s fournissant un signal G(t) représentatif de l'évolution, en fonction du temps t, de la glycémie du patient. Le modèle 501 de la figure 5 est un modèle de type boîte noire, c'est-à-dire un modèle non physiologique, défini par la seule observation des effets des variables d'entrée EE(t), IOB(t) et COB(t) sur la sortie G(t), sans tenir compte des différents mécanismes physiologiques connus s'opérant dans le corps du patient et ayant un effet sur la glycémie du patient. Le modèle 501 de la figure 5 est par exemple un modèle de type ARX, c'est-à-dire un modèle autorégressif à entrées externes. A titre d'exemple, le modèle 501 est défini par une équation unique, comme suit :

$$G(t) = a.y_G + b.u_{EE} + c.u_{IOB} + d.u_{COB} \qquad (eq2)$$

où t est une variable temps discrétisée, $a = [a_1, ..., a_{na}]$ est un vecteur de paramètres de dimension $n_a$, avec $n_a$ entier supérieur ou égal à 1 et de préférence supérieur ou égal à 2, $b = [b_1, ..., b_{nb}]$ est un vecteur de paramètres de dimension $n_b$, avec $n_b$ entier supérieur ou égal à 1 et de préférence supérieur ou égal à 2, $c = [c_1, ..., c_{nc}]$ est un vecteur de paramètres de dimension $n_c$, avec $n_c$ entier supérieur ou égal à 1 et de préférence supérieur ou égal à 2, $d = [d_1, ..., d_{nd}]$ est un vecteur de paramètres de dimension $n_d$, avec $n_d$ entier supérieur ou égal à 1 et de préférence supérieur ou égal à 2, $y_G = [G(t-1), ..., G(t-n_a)]$ est un vecteur de régression de dimension $n_a$, $u_{EE} = [EE(t-n_{k1}-1), ..., EE(t-n_{k1}-n_b)]$ est un vecteur de régression de dimension $n_b$, avec $n_{k1}$ entier supérieur ou égal à 1, $u_{IOB} = [IOB(t-n_{k2}-1), ..., IOB(t-n_{k2}-n_c)]$ est un vecteur de régression de dimension $n_c$, avec $n_{k2}$ entier supérieur ou égal à 1, et $u_{COB} = [COB(t-n_{k3}-1), ..., COB(t-n_{k3}-n_d)]$ est un vecteur de régression de dimension $n_d$, avec $n_{k3}$ entier supérieur ou égal à 1.

Si l'on désigne par T la période d'échantillonnage du système, pour chacune des variables G, EE, IOB et COB du modèle, la valeur G(t), respectivement EE(t), respectivement IOB(t), respectivement COB(t) correspond à la valeur de la variable à l'instant t, et la valeur G(t-j), respectivement EE(t-j), respectivement IOB(t-j), respectivement COB(t-j), avec j entier, correspond à la valeur de la variable à l'instant t-j*T.

A titre d'exemple, la période d'échantillonnage du système est comprise dans la plage allant de 0,5 à 30 minutes, par exemple de l'ordre de 10 minutes.

Dans cet exemple, les grandeurs $n_a$, $n_b$, $n_c$ et $n_d$ sont les ordres du modèle, et les grandeurs $n_{k1}$, $n_{k2}$ et $n_{k3}$ sont les délais du modèle.

La variable d'entrée IOB du modèle est calculée à partir des données d'insuline injectée I(t) et d'une fonction d'action $h_{IOB}$ représentant l'évolution, en fonction du temps, de l'effet de l'insuline injectée sur l'absorption du glucose sanguin. La variable d'entrée IOB du modèle est définie comme suit:

$$IOB(t) = \sum_{k=0}^{K} I(t-k) * h_{IOB}(k) \qquad (eq3)$$

où K est un entier supérieur à 1 et k est un entier allant de 0 à K.

La variable d'entrée COB du modèle est calculée à partir des données de glucose ingéré CHO(t) et d'une fonction d'action $h_{COB}$ représentant l'évolution, en fonction du temps, de l'effet du glucose ingéré sur la glycémie (ou taux de glucose sanguin). La variable d'entrée COB du modèle est définie comme suit :

$$COB(t) = \sum_{k=0}^{K} CHO(t-k) * h_{COB}(k) \qquad (eq4)$$

Dans les équations eq3 et eq4 susmentionnées, la grandeur K définit la durée d de prise en compte de l'historique d'insuline injectée ou de glucose ingéré pour le calcul des variables d'entrée IOB(t) et COB(t) du modèle, telle que d = K*T. A titre d'exemple, la durée d est comprise entre 500 et 2500 minutes, par exemple de l'ordre de 1500 minutes, par exemple égale à 1440 minutes.

Les fonctions d'action $h_{IOB}$ et $h_{COB}$ sont par exemple définies comme suit :

$$h_{IOB}(t) = \left[1 + \frac{t}{\tau_{IOB}}\right] e^{-\frac{t}{\tau_{IOB}}} \qquad (eq5)$$

$$h_{COB}(t) = \left[1 + \frac{t}{\tau_{COB}}\right] e^{-\frac{t}{\tau_{COB}}} \qquad (eq6)$$

où $\tau_{IOB}$ est une constante de temps, par exemple comprise entre 5 et 120 minutes, par exemple de l'ordre de 50 minutes, et où $\tau_{COB}$ est une constante de temps, par exemple différente de $\tau_{IOB}$, par exemple comprise entre 5 et 120 minutes, par exemple de l'ordre de 40 minutes.

La variable d'entrée EE(t) du modèle peut être calculée à partir des signaux de sortie du capteur de mouvements 405 et du capteur de rythme cardiaque 407 (figure 4) du système. A titre d'exemple, le capteur de mouvements 405 fournit un signal $S_{CPM}$ représentatif des mouvements effectués par le patient, par exemple représentatif d'un nombre de mouvements par minute effectués par le patient, par exemple représentatif du nombre de pas par minute effectués par le patient, et le capteur de rythme cardiaque 407 fournit un signal $S_{HR}$ représentatif du rythme cardiaque (nombre de pulsations par minute) du patient. Plus particulièrement, dans cet exemple, le signal $S_{HR}$ est égal à la différence entre le rythme cardiaque (nombre de pulsations par minute) du patient à l'instant t et le rythme cardiaque au repos du patient (qui peut être une constante du système). La variable d'entrée EE(t) peut alors être calculée comme suit :

$$EE(t) = \begin{cases} \alpha_1 * S_{HR}(t) + \beta_1, & S_{HR} \geq S1 \\ \alpha_2 * S_{LC}(t) + \beta_2, & S_{HR} < S1 \ et \ S_{LC} < S2 \\ \alpha_3 * S_{LC}(t) + \beta_3, & S_{HR} < S1 \ et \ S_{LC} \geq S2 \end{cases} \qquad (eq7)$$

où le signal $S_{LC}$ est une combinaison linéaire des signaux $S_{HR}$ et $S_{CPM}$, telle que $S_{LC}(t) = \theta1*s_{CPM}(t)+\theta2*s_{HR}(t)$, et où les grandeurs $\alpha_1$, $\alpha_2$, $\alpha_3$, $\beta_1$, $\beta_2$, $\beta_3$, $\theta1$, $\theta2$, S1 et S2 sont des paramètres du système.

A titre d'exemple, les signaux EE(t), IOB(t) et COB(t) peuvent être calculés à une première période d'échantillonnage, par exemple de l'ordre de 1 minute, puis échantillonnés à une période T plus élevée, par exemple de l'ordre de 10 minutes.

A titre d'exemple, la valeur $\alpha_1$ est comprise entre 1 et 10, par exemple de l'ordre de 5,45, la valeur $\alpha_2$ est comprise entre 100 et 500, par exemple de l'ordre de 256,1, la valeur $\alpha_3$ est comprise entre 10 et 200, par exemple de l'ordre de 86, la valeur $\beta_1$ est comprise entre -200 et 0, par exemple de l'ordre de -66,1, la valeur $\beta_2$ est comprise entre -10 et 10, par exemple de l'ordre de -0,13, et la valeur $\beta_3$ est comprise entre 10 et 200, par exemple de l'ordre de 82,4. A titre d'exemple, le seuil S1 est compris entre 20 et 60, par exemple de l'ordre de 40, et le seuil S2 est compris entre 0,1 et 1, par exemple de l'ordre de 0,5. Les valeurs $\theta1$ et $\theta2$ sont par exemple respectivement de l'ordre de 0,000084 et de l'ordre de 0,0086. Un exemple de procédé d'estimation de la dépense énergétique d'un individu, pouvant être utilisé pour calculer la variable d'entrée EE(t) du modèle, est décrit plus en détail dans l'article intitulé "An original piecewise model for computing energy expenditure from accelerometer and heart rate signals" de H.M. Romero-Ugalde et al. (Physiological Measurement, vol. 38, no. 8, p. 1599, 2017).

La figure 6 est un diagramme illustrant un exemple d'un procédé de construction du modèle de la figure 5. Plus particulièrement, étant donnée la structure de modèle décrite ci-dessus et définie par l'équation eq2, la figure 6 illustre un exemple de procédé de dimensionnement du modèle, c'est-à-dire de détermination des ordres $n_a$, $n_b$, $n_c$ et $n_d$ et des délais $n_{k1}$, $n_{k2}$ et $n_{k3}$ du modèle. Pour dimensionner le modèle, on utilise ici une base de données contenant, pour un ou plusieurs patients, un historique de l'activité physique PA(t) (par exemple déterminé à partir d'un historique des données de sortie d'un accéléromètre porté par le patient et d'un capteur du rythme cardiaque du patient), des prises de glucose CHO(t), des prises d'insuline I(t), et de la glycémie réelle (mesurée par un capteur de glycémie) $G^r(t)$ du patient.

Le procédé de la figure 6 comprend une étape 601 au cours de laquelle on affecte, par exemple arbitrairement, une valeur à chacune des dimensions $n_a$, $n_b$, $n_c$, $n_d$, $n_{k1}$, $n_{k2}$ et $n_{k3}$ du modèle.

Le procédé de la figure 6 comprend ensuite une étape 603 de calcul des paramètres du modèle, à savoir les valeurs $a_1$, ..., $a_{na}$, $b_1$, ..., $b_{nb}$, $c_1$, ..., $c_{nc}$, $d_1$, ..., $d_{nd}$. Lors de cette étape, la glycémie du patient est estimée à partir du modèle sur une période d'observation passée, en tenant compte de l'historique d'injection d'insuline, de prise de glucose, et d'activité physique, puis la courbe de glycémie G estimée par le modèle est comparée à la courbe de glycémie réelle $G^r$ mesurée par le capteur pendant cette même période. On recherche alors un jeu de paramètres $a_1$, ..., $a_{na}$, $b_1$, ..., $b_{nb}$, $c_1$, ..., $c_{nc}$, $d_1$, ..., $d_{nd}$ conduisant à minimiser une grandeur représentative de l'erreur entre la courbe de glycémie estimée par le modèle et la courbe de glycémie réelle pendant la période d'observation. A titre d'exemple, on recherche un jeu de paramètres conduisant à minimiser un indicateur représentatif de l'aire entre la courbe de glycémie estimée par le modèle et la courbe de glycémie réelle pendant la période d'observation, aussi appelé écart quadratique moyen entre la glycémie estimée et la glycémie réelle, par exemple identique ou similaire à l'indicateur m de l'équation eq1 susmentionnée. L'algorithme de recherche de paramètres optimaux utilisé lors de cette étape n'est pas détaillé dans la présente demande, les modes de réalisation décrits étant compatibles avec les algorithmes usuels utilisés dans des domaines variés pour résoudre des problèmes d'optimisation de paramètres par minimisation d'une fonction de coût, par exemple par la méthode des moindres carrés. On notera que dans le cas d'une base de données comportant les données d'historique de plusieurs patients, l'étape 603 peut comprendre les étapes successives suivantes :

- détermination d'une matrice de régression pour chaque patient à partir des données d'historique du patient ;
- concaténation des matrices de régression des différents patients ; et
- détermination des paramètres optimaux du modèle conduisant à minimiser l'erreur entre la courbe de glycémie estimée par le modèle et la courbe de glycémie réelle.

Le procédé de la figure 6 comprend en outre, après l'étape 603, une fois les paramètres optimaux (pour les dimensions fixées à l'étape 601) du modèle déterminés, une étape 605 de détermination de l'erreur entre la courbe de glycémie estimée par le modèle et la courbe de glycémie réelle, sur la période d'observation passée considérée. A titre d'exemple, lors de cette étape, on mémorise l'indicateur d'erreur calculé à l'étape 603 pour le jeu de paramètres considéré.

Le procédé de la figure 6 est réitéré une pluralité de fois en modifiant à chaque fois une ou plusieurs dimensions (ordre ou délai) du modèle. A titre d'exemple, l'ordre $n_a$ est modifié de façon à balayer toutes les valeurs de la plage [1,15], l'ordre $n_b$ est modifié de façon à balayer toutes les valeurs de la plage [1,15], l'ordre $n_c$ est modifié de façon à balayer toutes les valeurs de la plage [1,15], l'ordre $n_d$ est modifié de façon à balayer toutes les valeurs de la plage [1,15], le délai $n_{k1}$ est modifié de façon à balayer toutes les valeurs de la plage [1,15], le délai $n_{k2}$ est modifié de façon à balayer toutes les valeurs de la plage [1,15], et le délai $n_{k3}$ est modifié de façon à balayer toutes les valeurs de la plage [1,15]. Ainsi, dans cet exemple, la suite d'étapes 601, 602 et 603 est réitérée N = 15*15*15*15*15*15*15 = 170859375 fois.

A l'issue des N itérations de la suite d'étapes 601, 602, 603, les dimensions et le jeu de paramètres correspondant, conduisant à la plus faible erreur entre la courbe de glycémie estimée par le modèle et la courbe de glycémie réelle, sur la période d'observation passée, sont sélectionnés. A titre d'exemple, les dimensions finales du modèle sélectionnées à l'issue du procédé de la figure 6 sont les suivantes : $n_a = 3$, $n_b = 5$, $n_c = 11$, $n_d = 7$, $n_{k1} = n_{k2} = n_{k3} = 2$.

Un avantage du modèle de type boîte noire décrit en relation avec la figure 5 est qu'il permet de réaliser des prédictions particulièrement fiables de l'évolution future de la glycémie du patient, ce que ne permettent pas les modèles physiologiques connus. En effet, s'il est bien connu que l'insuline favorise l'absorption du glucose présent dans le sang par différents organes du corps, et notamment par les muscles, et que la sensibilité à l'insuline et l'absorption du glucose sanguin par les muscles augmentent en présence d'une activité physique, la quantification des effets combinés de ces différentes variables est très difficile à réaliser. L'utilisation d'un modèle de type boîte noire ayant pour entrées la dépense énergétique, l'insuline embarquée, et le glucose embarqué, permet avantageusement d'obtenir des prédictions de glycémie particulièrement fiables, notamment pendant et après une activité physique.

Le système de prédiction de la figure 4 peut être utilisé pour de nombreuses applications.

A titre d'exemple, le système de la figure 4 peut être utilisé pour mettre en oeuvre un système automatisé de régulation de glycémie de type MPC tel que décrit en relation avec les figures 1 à 3, en remplaçant le modèle physiologique 201 de la figure 2 par le modèle boîte noire 501 de la figure 5.

A titre de variante, le système de prédiction de la figure 4 peut être utilisé pour mettre en oeuvre un système automatisé de régulation de glycémie de type MPC basé sur un modèle physiologique, tel que décrit en relation avec les figures 1 à 3, le modèle boîte noire 501 de la figure 5 étant utilisé en redondance du modèle physiologique 201, pour contrôler la fiabilité des prédictions réalisées à partir du modèle physiologique.

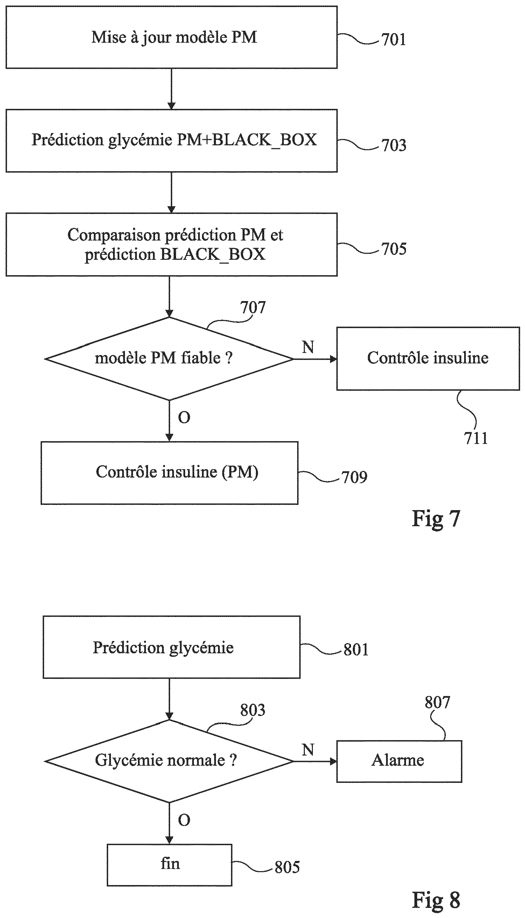

La figure 7 un diagramme illustrant un exemple d'un procédé automatisé de régulation de glycémie de type MPC basé sur un modèle physiologique, tel que décrit en relation avec les figures 1 à 3, dans lequel le modèle boîte noire 501 de la figure 5 est utilisé pour contrôler la fiabilité des prédictions réalisées à partir du modèle physiologique.

Le procédé de la figure 7 comprend une étape 701 de re-calibration ou mise à jour du modèle physiologique, identique ou similaire à l'étape 301 du procédé de la figure 3. Lors de cette étape, l'unité de traitement et de contrôle 401 met en oeuvre un procédé de ré-estimation des paramètres temps-dépendants du modèle physiologique en tenant compte des données d'insuline effectivement injectée par le dispositif 103 et des données de glycémie réelle mesurée par le capteur 101 pendant une période d'observation passée. Plus particulièrement, lors de l'étape de calibration, l'unité de traitement et de contrôle 401 simule le comportement du patient sur la période d'observation passée à partir du modèle physiologique (en tenant compte des éventuelles ingestions de glucose et injections d'insuline pendant cette période), et compare la courbe de glycémie estimée par le modèle à la courbe de glycémie réelle mesurée par le capteur pendant cette même période. L'unité de traitement et de contrôle 401 recherche alors, pour les paramètres temps-dépendants du modèle, un jeu de valeurs conduisant à minimiser une grandeur représentative de l'erreur entre la courbe de glycémie estimée par le modèle et la courbe de glycémie réelle pendant la période d'observation. Lors de l'étape 701, outre les paramètres temps-dépendants du modèle physiologique, l'unité de traitement et de contrôle 401 peut définir un vecteur d'états initiaux des variables d'état du modèle.

Le procédé de la figure 7 comprend en outre, après l'étape 701, une étape 703 de prédiction, par l'unité de traitement et de contrôle 401, de l'évolution temporelle de la glycémie du patient sur une période de prédiction à venir de durée $T_{pred}$. Lors de cette étape, deux courbes de glycémie à venir G1 et G2 sont déterminées, la première (la courbe G1) correspondant à la prédiction de glycémie réalisée à partir du modèle physiologique sur la période de prédiction à venir, de façon identique ou similaire à ce qui a été décrit en relation avec la figure 3, et la deuxième (la courbe G2) correspondant à la prédiction de glycémie réalisée à partir du modèle boîte noire de la figure 5 sur la même période de prédiction à venir.

Le procédé de la figure 7 comprend de plus, après l'étape 703, une étape 705 de comparaison entre la courbe de glycémie à venir G1 estimée à partir du modèle physiologique et la courbe de glycémie à venir G2 estimée à partir du modèle boîte noire. A titre d'exemple, lors de cette étape, l'unité de traitement et de contrôle 401 calcule un ou plusieurs indicateurs numériques représentatifs de l'écart entre les courbes de glycémie prédites G1 et G2, par exemple l'écart quadratique moyen entre les courbes de glycémie prédites G1 et G2.

Le procédé de la figure 7 comprend en outre, après l'étape 705, une étape 707 de vérification de la fiabilité du modèle physiologique mis à jour à l'étape 701. Plus particulièrement, lors de cette étape, la fiabilité du modèle physiologique est considérée comme suffisante par l'unité de traitement et de contrôle 401 lorsque la (les) valeur(s) du ou des indicateurs d'écart entre la courbe de glycémie à venir G1 estimée à partir du modèle physiologique et la courbe de glycémie à venir G2 estimée à partir du modèle boîte noire est(sont) inférieure(s) à un(des) seuil(s) prédéfini(s), et insuffisante dans le cas contraire.

Si le modèle physiologique est considéré comme suffisamment fiable à l'étape 707 (O), une étape 709 est mise en oeuvre, au cours de laquelle l'unité de traitement et de contrôle 401 détermine, en tenant compte de la courbe de glycémie future G1 estimée à partir du modèle physiologique, des doses d'insuline à injecter au patient pendant la période de prédiction à venir. A l'issue de cette étape, l'unité de traitement et de contrôle 401 peut programmer le dispositif d'injection 103 pour administrer les doses déterminées pendant la période de prédiction à venir.

Si le modèle physiologique est jugé insuffisamment fiable à l'étape 707 (N), l'unité de traitement et de contrôle 401 cesse d'utiliser le modèle physiologique pour réguler l'administration de l'insuline au patient, et met en oeuvre une méthode de régulation de substitution lors d'une étape 711. A titre d'exemple, lors de l'étape 711, l'unité de traitement et de contrôle 401 utilise la courbe de glycémie future G2 estimée à partir du modèle boîte noire de la figure 5 pour déterminer les doses d'insuline à injecter au patient pendant la période de prédiction à venir. A titre de variante, lors de l'étape 711, l'unité de traitement et de contrôle 401 utilise un modèle physiologique simplifié, par exemple un modèle compartimentai comportant un nombre de variables d'état et un nombre de paramètres réduits par rapport au modèle initial, pour prédire l'évolution de la glycémie du patient et réguler en conséquence l'injection d'insuline. A titre de variante, lors de l'étape 711, l'unité de traitement et de contrôle 401 cesse de mettre en oeuvre une commande prédictive, c'est-à-dire qu'elle cesse d'utiliser un modèle mathématique pour prédire la glycémie future du patient et réguler en conséquence l'injection d'insuline. Dans ce cas, l'unité de traitement et de contrôle 401 commande par exemple le dispositif d'injection d'insuline 103 pour administrer des doses préprogrammées d'insuline, correspondant par exemple à un débit basal de référence prescrit au patient. Alternativement, l'unité de traitement et de contrôle 401 utilise un algorithme de type matrice décisionnelle pour déterminer les doses d'insuline à administrer au patient, en fonction de divers paramètres observés tels que le niveau courant de glycémie mesuré par le capteur 101, ou encore la vitesse de variation (ou pente) de la glycémie sur une période passée.

Les étapes 701, 703, 705, 707 et, le cas échéant, 709 ou 711, sont par exemple réitérées périodiquement, par exemple toutes les 1 à 20 minutes, et/ou à chaque nouvelle ingestion de glucose signalée par le patient, et/ou à chaque nouvelle administration d'une dose d'insuline par le dispositif d'injection 103.

La figure 8 est un diagramme illustrant un autre exemple d'un procédé automatisé de contrôle de glycémie pouvant être mis en oeuvre par le système de la figure 4.

Dans cet exemple, l'unité de traitement et de contrôle 401 peut mettre en oeuvre un procédé de régulation de glycémie quelconque, par exemple un procédé de régulation en boucle fermée du type décrit en relation avec les figures 1 à 3, ou un procédé de régulation en boucle ouverte dans lequel le patient définit lui-même les doses d'insuline à injecter sur la base de son taux de glycémie, de son historique d'injection d'insuline, et de son historique de prise de repas.

Dans l'exemple de la figure 8, le modèle boîte noire de la figure 5 est utilisé par l'unité de traitement et de contrôle 401 pour prédire l'évolution future de la glycémie du patient, et déclencher une alarme, par exemple une alarme sonore et/ou visuelle, lorsque le modèle prédit que la glycémie du patient va sortir d'une plage souhaitée, par exemple s'il existe un risque que le patient tombe en hypoglycémie.

Le procédé de la figure 8 comprend une étape 801 de prédiction, par l'unité de traitement et de contrôle 401, à partir du modèle boîte noire de la figure 5, de l'évolution temporelle de la glycémie du patient sur une période de prédiction à venir.

Le procédé de la figure 8 comprend en outre, après l'étape 801, une étape 803 au cours de laquelle l'unité de traitement et de contrôle 401 détermine si la glycémie à venir prédite par le modèle de la figure 5 présente ou non un risque pour le patient. Pour cela, l'unité de traitement et de contrôle 401 vérifie que la courbe de glycémie à venir prédite à l'étape 801 ne sort pas d'une plage souhaitée.

Si, à l'étape 803, la glycémie prédite est jugée acceptable (O), le procédé se termine lors d'une étape 805.

Si en revanche, à l'étape 803, la glycémie prédite est jugée inacceptable ou dangereuse pour le patient (N), l'unité de traitement et de contrôle 401 déclenche une alarme lors d'une étape 807, de façon à avertir le patient du risque d'hyperglycémie ou d'hypoglycémie, par exemple pour lui recommander de cesser toute activité physique et/ou de consommer du sucre en cas de risque d'hypoglycémie.

Les étapes 801, 803 et, le cas échéant, 805 ou 807, sont par exemple réitérées périodiquement.

Des modes de réalisation particuliers ont été décrits. Diverses variantes et modifications apparaîtront à l'homme de l'art. En particulier, les modes de réalisation décrits ne se limitent pas aux exemples d'utilisation du système de prédiction de glycémie de la figure 4 décrits en relation avec les figures 7 et 8. Plus généralement, le système de prédiction proposé, basé sur l'utilisation du modèle boîte noire de la figure 5, peut être utilisé pour toute application pouvant tirer profit d'une prédiction fiable de l'évolution future de la glycémie d'un patient.

De plus, les modes de réalisation décrits ne se limitent pas à l'exemple décrit ci-dessus dans lequel la variable d'entrée EE(t) du modèle est calculée en tenant compte d'un signal de sortie d'un capteur des mouvements du patient et d'un signal de sortie d'un capteur du rythme cardiaque du patient. A titre de variante, la variable d'entrée EE(t) peut être calculée en tenant compte uniquement du signal de sortie du capteur de mouvements ou uniquement du signal de sortie du capteur de rythme cardiaque. Plus généralement, la variable d'entrée EE(t) peut être constituée par tout signal ou combinaison de signaux représentatifs de l'activité physique du patient. A titre d'exemple, la variable d'entrée EE(t) peut être une combinaison d'un signal mesuré au moyen d'un capteur quelconque, par exemple le capteur de mouvements 405 et/ou le capteur de rythme cardiaque 407 de la figure 4, et d'un signal d'intensité d'activité physique déclaré par le patient au moyen d'une interface utilisateur (non détaillée). A titre de variante, la variable d'entrée EE(t) peut être calculée uniquement à partir d'un signal d'intensité d'activité déclaré par le patient.

Par ailleurs, les modes de réalisation décrits ne se limitent pas au cas particulier décrit ci-dessus dans lequel le modèle boîte noire utilisé pour prédire la glycémie future du patient est un modèle de type ARX. Plus généralement tout autre type de modèle boîte noire peut être utilisé, par exemple un modèle de type ARMAX (de l'anglais "Auto Régressive Moving Average with eXternal inputs" - modèle autorégressif à moyenne mobile avec des entrées externes), ou un modèle basé sur un réseau neuronal.

De plus, outre les variables d'entrée EE(t), IOB(t) et COB(t) décrites ci-dessus, le modèle de la figure 5 peut comprendre une variable d'entrée supplémentaire représentative de la variabilité du rythme cardiaque du patient, afin de tenir compte du stress dans la prédiction de glycémie réalisée à partir du modèle.

**Revendications**

1. Système automatisé de contrôle de la glycémie d'un patient, comportant une unité de traitement et de contrôle (401) adaptée à prédire, à partir d'un premier modèle mathématique (501), l'évolution future de la glycémie G(t) du patient sur une période de prédiction, dans lequel le premier modèle prend pour entrées :

une variable EE(t) représentative de l'évolution, en fonction du temps, de la dépense énergétique du patient ;
une variable IOB(t) représentative de l'évolution, en fonction du temps, de la quantité d'insuline embarquée du patient ; et
une variable COB(t) représentative de l'évolution, en fonction du temps, de la quantité de glucose embarquée du patient,
et dans lequel le premier modèle mathématique (501) est un modèle de type boîte noire, c'est-à-dire un modèle non physiologique défini par la seule observation des effets des variables d'entrée EE(t), IOB(t) et COB(t) sur la variable de sortie G(t), sans tenir compte des différents mécanismes physiologiques connus s'opérant dans le corps du patient,
dans lequel, en fonctionnement, l'unité de traitement et de contrôle (401) reçoit un signal CHO(t) représentatif de l'évolution, en fonction du temps, de la quantité de glucose ingérée par le patient, et un signal I (t) représentatif de l'évolution, en fonction du temps, de la quantité d'insuline injectée au patient, et dans lequel l'unité de traitement et de contrôle (401) est configurée pour calculer les variables d'entrée IOB(t) et COB(t) du premier modèle (501) comme suit :

$$IOB(t) = \sum_{k=0}^{K} I(t-k) * h_{IOB}(k),$$

et

$$COB(t) = \sum_{k=0}^{K} CHO(t-k) * h_{COB}(k),$$

où K est un entier supérieur à 1, k est un entier allant de 0 à K, $h_{IOB}$ est une fonction d'action représentant l'évolution, en fonction du temps, de l'effet de l'insuline injectée sur l'absorption du glucose sanguin, et $h_{COB}$ est une fonction d'action représentant l'évolution, en fonction du temps, de l'effet du glucose ingéré sur la glycémie.

2. Système selon la revendication 1, dans lequel le premier modèle mathématique (501) est défini comme suit :

$$G(t) = a.y_G + b.u_{EE} + c.u_{IOB} + d.u_{COB},$$

où t est une variable temps discrétisée, a = [$a_1$, ..., $a_{na}$] est un vecteur de paramètres de dimension $n_a$, avec $n_a$ entier supérieur ou égal à 1, b = [$b_1$, ..., $b_{nb}$] est un vecteur de paramètres de dimension $n_b$, avec $n_b$ entier supérieur ou égal à 1, c = [$c_1$, ..., $c_{nc}$] est un vecteur de paramètres de dimension $n_c$, avec $n_c$ entier supérieur ou égal à 1, d = [$d_1$, ..., $d_{nd}$] est un vecteur de paramètres de dimension $n_d$, avec $n_d$ entier supérieur ou égal à 1, $y_G$ = [G(t-1), ..., G(t-$n_a$)] est un vecteur de régression de dimension $n_a$, $u_{EE}$ = [EE(t-$n_{k1}$), ..., EE(t-$n_{k1}$-$n_b$)] est un vecteur de régression de dimension $n_b$, avec $n_{k1}$ entier supérieur ou égal à 1, $u_{IOB}$ = [IOB(t-$n_{k2}$), ..., IOB(t-$n_{k2}$-$n_c$)] est un vecteur de régression de dimension $n_c$, avec $n_{k2}$ entier supérieur ou égal à 1, et $u_{COB}$ = [COB (t-$n_{k3}$) , ..., COB(t-$n_{k3}$-$n_d$)] est un vecteur de régression de dimension $n_d$, avec $n_{k3}$ entier supérieur ou égal à 1.

3. Système selon la revendication 2, dans lequel $n_a$ est compris dans la plage [1,15], $n_b$ est compris dans la plage [1,15], $n_c$ est compris dans la plage [1,15], $n_d$ est compris dans la plage [1,15], $n_{k1}$ est compris dans la plage [1,15], $n_{k2}$ est compris dans la plage [1,15], et $n_{k3}$ est compris dans la plage [1,15] .

4. Système selon l'une quelconque des revendications 1 à 3, dans lequel les fonctions d'actions $h_{IOB}$ et $h_{COB}$ sont définies comme suit :

$$h_{IOB}(t) = \left[1 + \frac{t}{\tau_{IOB}}\right] e^{-\frac{t}{\tau_{IOB}}},$$

et

$$h_{COB}(t) = \left[1 + \frac{t}{\tau_{COB}}\right] e^{-\frac{t}{\tau_{COB}}},$$

où $\tau_{IOB}$ et $\tau_{COB}$ sont des constantes de temps.

**5.** Système selon l'une quelconque des revendications 1 à 4, comprenant en outre un dispositif (403) de mesure d'une activité physique du patient, la variable d'entrée EE(t) du premier modèle (501) étant calculée par l'unité de traitement et de contrôle (401) à partir de données de sortie PA(t) du dispositif de mesure (403) .

**6.** Système selon la revendication 5, dans lequel le dispositif de mesure (403) comprend un capteur (405) de mouvements du patient.

**7.** Système selon la revendication 6, dans lequel le dispositif de mesure (403) comprend en outre un capteur (407) du rythme cardiaque du patient.

**8.** Système selon la revendication 7, dans lequel le capteur de mouvements (405) fournit un signal $S_{CPM}$ représentatif de mouvements effectués par le patient, et le capteur de rythme cardiaque (407) fournit un signal $S_{HR}$ représentatif du rythme cardiaque du patient, et dans lequel la variable d'entrée EE(t) du premier modèle (501) est calculée comme suit par l'unité de traitement et de contrôle (401) :

$$EE(t) = \begin{cases} \alpha_1 * S_{HR}(t) + \beta_1, & S_{HR} \geq S1 \\ \alpha_2 * S_{LC}(t) + \beta_2, & S_{HR} < S1 \, et \, S_{LC} < S2, \\ \alpha_3 * S_{LC}(t) + \beta_3, & S_{HR} < S1 \, et \, S_{LC} \geq S2 \end{cases}$$

où le signal $S_{LC}$ est une combinaison linéaire des signaux $S_{HR}$ et $S_{CPM}$, et les grandeurs $\alpha_1$, $\alpha_2$, $\alpha_3$, $\beta_1$, $\beta_2$, $\beta_3$, S1 et S2 sont des paramètres du système.

**9.** Système selon l'une quelconque des revendications 1 à 8, dans lequel l'unité de traitement et de contrôle (401) est configurée pour prédire, à partir du premier modèle mathématique (501), l'évolution future de la glycémie G(t) du patient sur une période de prédiction, déterminer si la glycémie G(t) prédite par le premier modèle reste ou non contenue dans une plage souhaitée, et déclencher une alarme si la glycémie G (t) prédite par le premier modèle sort de la plage souhaitée.

**10.** Système selon l'une quelconque des revendications 1 à 9, comportant en outre un capteur de glycémie (101) , et un dispositif d'injection d'insuline (103),

dans lequel l'unité de traitement et de contrôle (401) est adaptée à prédire l'évolution future de la glycémie du patient à partir d'un deuxième modèle mathématique (201) et à commander le dispositif d'injection d'insuline (103) en tenant compte de cette prédiction,
l'unité de traitement et de contrôle (401) étant configurée pour :

a) mettre en oeuvre une étape de calibration automatique du deuxième modèle (201) en tenant compte d'un historique de glycémie mesurée par le capteur (101) au cours d'une période d'observation passée ;
b) à l'issue de l'étape de calibration, réaliser une première prédiction de l'évolution future de la glycémie du patient à partir du premier modèle (501) et, sur la même période de prédiction, une deuxième prédiction de l'évolution future de la glycémie du patient à partir du deuxième modèle (201) ;
c) calculer au moins un indicateur numérique représentatif de l'écart entre les première et deuxième prédictions ; et
d) en fonction de la valeur de l'indicateur numérique, commander le dispositif d'injection d'insuline (103) en tenant compte de la deuxième prédiction ou commander le dispositif d'injection d'insuline (103) sans tenir compte de la deuxième prédiction.

**11.** Système selon la revendication 10, dans lequel le deuxième modèle (201) est un modèle physiologique comportant un système d'équations différentielles décrivant l'évolution d'une pluralité de variables d'état en fonction du temps.

12. Système selon la revendication 11, dans lequel l'étape a) de calibration automatique du deuxième modèle (201) comprend une étape d'estimation de paramètres du système d'équations différentielles par minimisation d'une grandeur représentative de l'erreur, pendant la période d'observation passée, entre la glycémie estimée à partir du deuxième modèle physiologique et la glycémie mesurée par le capteur (101).

**Patentansprüche**

1. Ein automatisiertes System zum Steuern eines Blutzuckerspiegels eines Patienten, das eine Verarbeitungs- und Steuereinheit (401) umfasst, die angepasst ist, um, basierend auf einem ersten mathematischen Modell (501), die zukünftige Entwicklung des Blutzuckerspiegels G(t) des Patienten über eine Vorhersageperiode vorherzusagen, wobei das erste Modell als Eingaben verwendet:

eine Variable EE(t), welche die zeitliche Entwicklung eines Energieverbrauchs des Patienten darstellt;
eine Variable IOB(t), welche die zeitliche Entwicklung einer vom Patienten mitgeführten Insulinmenge darstellt; und
eine Variable COB(t), welche die zeitliche Entwicklung einer vom Patienten mitgeführten Kohlenhydratmenge darstellt,
und wobei das erste mathematische Modell (501) ein Modell vom Typ einer Blackbox ist, das heißt ein nichtphysiologisches Modell, das nur durch die Beobachtung der Auswirkungen der Eingangsvariablen EE(t), IOB(t) und COB(t) auf die Ausgangsvariable G(t) definiert ist, ohne die verschiedenen bekannten physiologischen Mechanismen, die im Körper des Patienten wirken, zu berücksichtigen,
wobei, im Betrieb, die Verarbeitungs- und Steuereinheit (401) ein Signal CHO(t) empfängt, welches die zeitliche Entwicklung der vom Patienten aufgenommenen Kohlenhydratmenge darstellt, und ein Signal I(t), welches die zeitliche Entwicklung der dem Patienten injizierten Insulinmenge darstellt, und wobei die Verarbeitungs- und Steuereinheit (401) konfiguriert ist, um die Eingangsvariablen IOB(t) und COB(t) des ersten Modells (501) wie folgt zu berechnen:

$$IOB(t) = \sum_{k=0}^{K} I(t-k) * h_{IOB}(k),$$

und

$$COB(t) = \sum_{k=0}^{K} CHO(t-k) * h_{COB}(k),$$

wobei K eine ganze Zahl größer als 1 ist, k eine ganze Zahl im Bereich von 0 bis K ist, $h_{IOB}$ eine Wirkungsfunktion ist, welche die zeitliche Entwicklung der Auswirkung des injizierten Insulins auf die Absorption von Blutzucker darstellt, und $h_{COB}$ eine Wirkungsfunktion ist, welche die zeitliche Entwicklung der Auswirkung der aufgenommenen Kohlenhydrate auf den Blutzuckerspiegel darstellt.

2. System gemäß Anspruch 1, wobei das erste mathematische Modell (501) wie folgt definiert ist:

$$G(t) = a.y_G + b.u_{EE} + c.u_{IOB} + d.u_{COB},$$

wobei t eine diskretisierte Zeitvariable ist, $a = [a_1, ..., a_{na}]$ ein Vektor von Parametern der Dimension $n_a$ ist, wobei $n_a$ eine ganze Zahl größer als oder gleich 1 ist, $b = [b_1, ..., b_{nb}]$ ein Vektor von Parametern der Dimension $n_b$ ist, wobei $n_b$ eine ganze Zahl größer als oder gleich 1 ist, $c = [c_1, ...., c_{nc}]$ ein Vektor von Parametern der Dimension $n_c$ ist, wobei $n_c$ eine ganze Zahl größer oder gleich 1 ist, $d = [d_1, ..., d_{nd}]$ ein Vektor von Parametern der Dimension $n_d$ ist, wobei $n_d$ eine ganze Zahl größer als oder gleich 1 ist, $y_G = [G(t-1), ..., G(t-n_a)]$ ein Regressionsvektor der Dimension $n_a$ ist, $u_{EE} = [EE(t-n_{k1}), ..., EE(t-n_{k1}-n_b)]$ ein Regressionsvektor der Dimension $n_b$ ist, wobei $n_{k1}$ eine ganze Zahl größer als oder gleich 1 ist, $u_{IOB} = [IOB(t-n_{k2}), ... , IOB(t-n_{k2}-n_c)]$ ein Regressionsvektor der Dimension $n_c$ ist, wobei $n_{k2}$ eine ganze Zahl größer als oder gleich 1 ist, und $u_{COB} = [COB(t-n_{k3}), ..., COB(t-n_{k3}-n_d)]$ ein Regressionsvektor der Dimension $n_d$ ist, wobei $n_{k3}$ eine ganze Zahl größer als oder gleich 1 ist.

3. System gemäß Anspruch 2, wobei $n_a$ im Bereich [1,15] liegt, $n_b$ im Bereich [1,15] liegt, $n_c$ im Bereich [1,15] liegt, $n_d$ im Bereich [1,15] liegt, $n_{k1}$ im Bereich [1,15] liegt, $n_{k2}$ im Bereich [1,15] liegt und $n_{k3}$ im Bereich [1,15] liegt.

4. System gemäß irgendeinem der Ansprüche 1 bis 3, wobei die Wirkungsfunktionen $h_{IOB}$ und $h_{COB}$ wie folgt definiert sind:

$$h_{IOB}(t) = \left[1 + \frac{t}{\tau_{IOB}}\right] e^{-\frac{t}{\tau_{IOB}}},$$

und

$$h_{COB}(t) = \left[1 + \frac{t}{\tau_{COB}}\right] e^{-\frac{t}{\tau_{COB}}},$$

wobei $\tau_{IOB}$ und $\tau_{COB}$ zeitliche Konstanten sind.

5. System gemäß irgendeinem der Ansprüche 1 bis 4, ferner umfassend eine Vorrichtung (403) zum Messen einer körperlichen Aktivität des Patienten, wobei die Eingangsvariable EE(t) des ersten Modells (501) durch die Verarbeitungs- und Steuereinheit (401) basierend auf Ausgangsdaten PA(t) der Messvorrichtung (403) berechnet wird.

6. System gemäß Anspruch 5, wobei die Messvorrichtung (403) einen Sensor (405) zum Erfassen von Bewegungen des Patienten umfasst.

7. System gemäß Anspruch 6, wobei die Messvorrichtung (403) ferner einen Sensor (407) zum Erfassen einer Herzfrequenz des Patienten umfasst.

8. System gemäß Anspruch 7, wobei der Bewegungssensor (405) ein Signal $S_{CPM}$ liefert, welches die vom Patienten ausgeführten Bewegungen darstellt, und der Herzfrequenzsensor (407) ein Signal $S_{HR}$ liefert, welches die Herzfrequenz des Patienten darstellt, und wobei die Eingangsvariable EE(t) des ersten Modells (501) wie folgt durch die Verarbeitungs- und Steuereinheit (401) berechnet wird:

$$EE(t) = \begin{cases} \alpha_1 * S_{HR}(t) + \beta_1, & S_{HR} \geq S1 \\ \alpha_2 * S_{LC}(t) + \beta_2, & S_{HR} < S1 \, et \, S_{LC} < S2, \\ \alpha_3 * S_{LC}(t) + \beta_3, & S_{HR} < S1 \, et \, S_{LC} \geq S2 \end{cases}$$

wobei das Signal $S_{LC}$ eine Linearkombination der Signale $S_{HR}$ und $S_{CPM}$ ist und die Größen $\alpha_1$, $\alpha_2$, $\alpha_3$, $\beta_1$, $\beta_2$, $\beta_3$, S1 und S2 Parameter des Systems sind.

9. System gemäß irgendeinem der Ansprüche 1 bis 8, wobei die Verarbeitungs- und Steuereinheit (401) konfiguriert ist, um basierend auf dem ersten mathematischen Modell (501) die zukünftige Entwicklung des Blutzuckerspiegels G(t) des Patienten über eine Vorhersageperiode vorherzusagen, um zu bestimmen, ob der durch das erste Modell vorhergesagte Blutzuckerspiegel G(t) innerhalb eines gewünschten Bereichs bleibt oder nicht darin liegt, und um einen Alarm auszulösen, wenn der durch das erste Modell vorhergesagte Blutzuckerspiegel G(t) den gewünschten Bereich verlässt.

10. System gemäß irgendeinem der Ansprüche 1 bis 9, wobei das System ferner einen Blutzuckersensor (101) und eine Insulininjektionsvorrichtung (103) umfasst,

wobei die Verarbeitungs- und Steuereinheit (401) angepasst ist, um die zukünftige Entwicklung des Blutzuckerspiegels des Patienten basierend auf einem zweiten mathematischen Modell (201) vorherzusagen und die Insulininjektionsvorrichtung (103) zu steuern, indem die Vorhersage berücksichtigt wird, und die Verarbeitungs- und Steuereinheit (401) konfiguriert ist zum:

a) Implementieren eines Schritts zur automatischen Kalibrierung des zweiten Modells (201), indem eine Historie des Blutzuckerspiegels berücksichtigt wird, welcher von dem Sensor (101) während einer vergangenen Beobachtungsperiode gemessen wurde;
b) am Ende des Kalibrierungsschritts, Vornehmen einer ersten Vorhersage der zukünftigen Entwicklung des Blutzuckerspiegels des Patienten basierend auf dem ersten Modell (501) und, über die gleiche Vorhersageperiode, einer zweiten Vorhersage der zukünftigen Entwicklung des Blutzuckerspiegels des Patienten basierend auf dem zweiten Modell (201);

c) Berechnen von zumindest einem numerischen Indikator, welcher die Abweichung zwischen der ersten und der zweiten Vorhersage darstellt; und

d) gemäß dem Wert des numerischen Indikators, Steuern der Insulininjektionsvorrichtung (103), indem die zweite Vorhersage berücksichtigt wird oder Steuern der Insulininjektionsvorrichtung (103) ohne die zweite Vorhersage zu berücksichtigen.

**11.** System gemäß Anspruch 10, wobei das zweite Modell (201) ein physiologisches Modell ist, das ein Differentialgleichungssystem umfasst, welches die zeitliche Entwicklung einer Vielzahl von Zustandsvariablen beschreibt.

**12.** System gemäß Anspruch 11, wobei der Schritt a) zur automatischen Kalibrierung des zweiten Modells (201) einen Schritt zur Schätzung von Parametern des Differentialgleichungssystems durch Minimierung einer Größe umfasst, welche den Fehler, während der vergangenen Beobachtungsperiode, zwischen dem Blutzuckerspiegel, der basierend auf dem physiologischen Modell geschätzt wird, und dem Blutzuckerspiegel, der durch den Sensor (101) gemessen wird, darstellt.

**Claims**

**1.** An automated system for controlling a patient's blood glucose, comprising a processing and control unit (401) capable of predicting, based on a first mathematical model (501), the future trend of the patient's blood glucose G(t) over a prediction period, wherein the first model takes as inputs:

a variable EE(t) representative of the time variation of the patient's energy expenditure;
a variable IOB(t) representative of the time variation of the patient's quantity of insulin on board; and
a variable COB(t) representative of the time variation of the patient's quantity of carbohydrate on board,
and wherein the first mathematical model (501) is a model of black box type, that is, a non-physiological model only defined by the observation of the effects of input variables EE(t), IOB(t) and COB(t) on output variable G(t), without taking into account the different known physiological mechanisms operating in the patient's body, wherein, in operation, the control and processing unit (401) receives a signal CHO(t), representative of the time variation of the quantity of carbohydrates ingested by the patient, and a signal I(t) representative of the time variation of the quantity of insulin injected to the patient, and wherein the control and processing unit (401) is configured to calculate the input variables IOB(t) and COB(t) of the first model (501) as follows:

$$IOB(t) = \sum_{k=0}^{K} I(t-k) * h_{IOB}(k),$$

and

$$COB(t) = \sum_{k=0}^{K} CHO(t-k) * h_{COB}(k),$$

where K is an integer greater than 1, k is an integer in the range from 0 to K, $h_{IOB}$ is an action function representing the time variation of the effect of the injected insulin on the absorption of blood glucose, and $h_{COB}$ is an action function representing the time variation of the effect of the ingested carbohydrates on the blood glucose.

**2.** The system according to claim 1, wherein the first mathematical model (501) is defined as follows:

$$G(t) = a.y_G + b.u_{EE} + c.u_{IOB} + d.u_{COB},$$

where t is a discretized time variable, $a = [a_1, ..., a_{na}]$ is a vector of parameters of dimension $n_a$, $n_a$ being an integer greater than or equal to 1, $b = [b_1, ..., b_{nb}]$ is a vector of parameters of dimension $n_b$, $n_b$ being an integer greater than or equal to 1, $c = [c_1, ..., c_{nc}]$ is a vector of parameters of dimension $n_c$, $n_c$ being an integer greater than or equal to 1, $d = [d_1, ..., d_{nd}]$ is a vector of parameters of dimension $n_d$, $n_d$ being an integer greater than or equal to 1, $y_G = [G(t-1), ..., G(t-n_a)]$ is a regression vector of dimension $n_a$, $u_{EE} = [EE(t-n_{k1}), ..., EE(t-n_{k1}-n_b)]$ is a regression vector of dimension $n_b$, $n_{k1}$ being an integer greater than or equal to 1, $u_{IOB} = [IOB(t-n_{k2}), ..., IOB(t-n_{k2}-n_c)]$ is a regression vector of dimension $n_c$, $n_{k2}$ being an integer greater than or equal to 1, and $u_{COB} = [COB(t-n_{k3}), ..., COB(t-n_{k3}-n_d)]$ is a regression vector of dimension $n_d$, $n_{k3}$ being an integer greater than or equal to 1.

3. The system according to claim 2, wherein $n_a$ is in range [1,15], $n_b$ is in range [1,15], $n_c$ is in range [1,15], $n_d$ is in range [1,15], $n_{k1}$ is in range [1,15], $n_{k2}$ is in range [1,15], and $n_{k3}$ is in range [1,15].

4. The system according to any of claims 1 to 3, wherein action functions $h_{IOB}$ and $h_{COB}$ are defined as follows:

$$h_{IOB}(t) = \left[1 + \frac{t}{\tau_{IOB}}\right] e^{-\frac{t}{\tau_{IOB}}},$$

and

$$h_{COB}(t) = \left[1 + \frac{t}{\tau_{COB}}\right] e^{-\frac{t}{\tau_{COB}}},$$

where $\tau_{IOB}$ and $\tau_{COB}$ are time constants.

5. The system according to any of claims 1 to 4, further comprising a device (403) for measuring a patient's physical activity, the input variable EE(t) of the first model (501) being calculated by the processing and control unit (401) based on output data PA(t) of the measurement device (403).

6. The system according to claim 5, wherein the measurement device (403) comprises a sensor (405) of the patient's movements.

7. The system according to claim 6, wherein the measurement device (403) further comprises a sensor (407) of the patient's heart rate.

8. The system according to claim 7, wherein the motion sensor (405) delivers a signal $S_{CPM}$ representative of movements performed by the patient, and the heart rate sensor (407) delivers a signal $S_{HR}$ representative of the patient's heart rate, and the input variable EE(t) of the first model (501) is calculated as follows by the processing and control unit (401):

$$EE(t) = \begin{cases} \alpha_1 * S_{HR}(t) + \beta_1, & S_{HR} \geq S1 \\ \alpha_2 * S_{LC}(t) + \beta_2, & S_{HR} < S1 \ et \ S_{LC} < S2, \\ \alpha_3 * S_{LC}(t) + \beta_3, & S_{HR} < S1 \ et \ S_{LC} \geq S2 \end{cases}$$

where signal $S_{LC}$ is a linear combination of signals $S_{HR}$ and $S_{CPM}$, and quantities $\alpha_1$, $\alpha_2$, $\alpha_3$, $\beta_1$, $\beta_2$, $\beta_3$, S1, and S2 are parameters of the system.

9. The system according to any of claims 1 to 8, wherein the processing and control unit (401) is configured to predict, based on the first mathematical model (501), the future trend of the patient's blood glucose G(t) over a prediction period, to determine whether the blood glucose G(t) predicted by the first model remains or not contained within a desired range, and to trigger an alarm if the blood glucose G(t) predicted by the first model comes out of the desired range.

10. The system according to any of claims 1 to 9, further comprising a blood glucose sensor (101), and an insulin injection device (103),

   wherein the processing and control unit (401) is capable of predicting the future trend of the patient's blood glucose based on a second mathematical model (201) and of controlling the insulin injection device (103) by taking the prediction into account, and
   the processing and control unit (401) being configured to:

a) implement a step of automatic calibration of the second model (201) by taking into account a history of the blood glucose measured by the sensor (101) during a past observation period;

b) at the end of the calibration step, make a first prediction of the future trend of the patient's blood glucose based on the first model (501) and, over the same prediction period, a second prediction of the future trend of the patient's blood glucose based on the second model (201);

c) calculate at least one numerical indicator representative of the deviation between the first and second predictions; and

d) according to the value of the numerical indicator, control the insulin injection device (103) by taking into account the second prediction or control the insulin injection device (103) without taking into account the second prediction.

11. The system according to claim 10, wherein the second mode (201) is a physiological model comprising a differential equation system describing the time variation of a plurality of state variables.

12. The system according to claim 11, wherein step a) of automatic calibration of the second model (201) comprises a step of estimation of parameters of the differential equation system by minimization of a quantity representative of the error, during the past observation period, between the blood glucose estimated based on the physiological model and the blood glucose measured by the sensor (101).

Fig 1

Fig 2

Fig 3

405   407

MVT   HR   403

101   CG

PA(t)

CTRL   CHO(t)

103   PMP   I(t)

401

**Fig 4**

501

EE(t) → e1

IOB(t) → e2   BLACK_BOX   s → G(t)

COB(t) → e3

**Fig 5**

Mise à jour ordres et délais   601

Calcul paramètres   603

Calcul erreur prédiction   605

**Fig 6**

```
┌─────────────────────────────────┐
│     Mise à jour modèle PM       │─── 701
└─────────────────────────────────┘
                │
                ▼
┌─────────────────────────────────┐
│ Prédiction glycémie PM+BLACK_BOX│─── 703
└─────────────────────────────────┘
                │
                ▼
┌─────────────────────────────────┐
│   Comparaison prédiction PM et  │─── 705
│      prédiction BLACK_BOX       │
└─────────────────────────────────┘
                │
                ▼         707
          ◇─────────────◇
         ╱               ╲        N   ┌──────────────────┐
        ◇  modèle PM fiable ? ◇──────▶│ Contrôle insuline │
         ╲               ╱            └──────────────────┘
          ◇─────────────◇                      │
                │ O                            711
                ▼
┌─────────────────────────────────┐
│     Contrôle insuline (PM)      │─── 709
└─────────────────────────────────┘
```

Fig 7

```
┌─────────────────────────────────┐
│     Prédiction glycémie         │─── 801
└─────────────────────────────────┘
                │
                ▼         803                 807
          ◇─────────────◇
         ╱               ╲        N   ┌──────────────────┐
        ◇ Glycémie normale ? ◇──────▶│      Alarme       │
         ╲               ╱            └──────────────────┘
          ◇─────────────◇
                │ O
                ▼
          ┌──────────┐
          │   fin    │─── 805
          └──────────┘
```

Fig 8

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 1852354
- FR 1658881
- FR 1658882
- FR 1756960
- US 2014066890 A

**Littérature non-brevet citée dans la description**

- **ROMAN HOVORKA et al.** Nonlinear model prédictive control of glucose concentration in subjects with type 1 diabetes. *Physiol Meas.,* 2004, vol. 25, 905-920
- **ROMAN HOVORKA et al.** Partitioning glucose distribution/transport, disposai, and endogenous production during IVGTT. *Am J Physiol Endocrinol Metab,* 2002, vol. 282, E992-E1007
- **H.M. ROMERO-UGALDE et al.** An original piecewise model for computing energy expenditure from accelerometer and heart rate signais. *Physiological Measurement,* 2017, vol. 38 (8), 1599